# EUROPEAN PATENT APPLICATION

(11) **EP 4 670 780 A1**
(43) Date of publication of application: **31.12.2025**
(21) Application number: 24777973.9
(22) Date of filing: 25.03.2024
(51) Int. Cl.: A61N 1/08

(54) **METHOD FOR CONTROLLING STIMULATOR, AND STIMULATOR, BRAIN-COMPUTER INTERFACE SYSTEM AND CHIP**

(30) Priority: 30.03.2023 CN 202310353226
(71) Applicant: Huawei Technologies Co., Ltd., Shenzhen, Guangdong 518129 (CN)
(72) Inventor: HUANG, Heming, Shenzhen, Guangdong 518129 (CN); CHEN, Yun, Shenzhen, Guangdong 518129 (CN); TIAN, Kun, Shenzhen, Guangdong 518129 (CN); LIAO, Jianxing, Shenzhen, Guangdong 518129 (CN); WANG, Kanwen, Shenzhen, Guangdong 518129 (CN); ZHANG, Ziyang, Shenzhen, Guangdong 518129 (CN)
(74) Representative: Körber, Martin Hans
(86) International application number: PCT/CN2024/083600
(87) International publication number: WO 2024/199185

(57) **Abstract**

Embodiments of this disclosure provide a method for controlling a stimulator, a stimulator, a brain-computer interface system, and a chip. The method includes: obtaining a voltage of an electrode coupled to the stimulator; comparing the obtained electrode voltage with a first threshold voltage during a period when the stimulator does not apply an electrical pulse to the electrode; if magnitude of the electrode voltage is not less than magnitude of the first threshold voltage, generating a first control signal to control a drive circuit of the stimulator to apply at least one electrical pulse to the electrode, where a charge polarity of the at least one electrical pulse is opposite to a charge polarity indicated by the electrode voltage; and after applying the at least one electrical pulse, generating a second control signal to connect the electrode to a reference potential. The solution of this disclosure can effectively improve safety of the stimulator.

## Description

### TECHNICAL FIELD

This disclosure relates to the field of electronic technologies, and more specifically, to a method for controlling a stimulator, a stimulator, and a brain-computer interface system.

### BACKGROUND

A brain-computer interface may establish a connection between a brain or a nervous system of organic life and a computing device or an electronic device, and is widely applied to many fields such as biomedicine and neurological rehabilitation. A stimulator is an important part of the brain-computer interface, and the stimulator may stimulate a biological tissue via an electrode to achieve a specific purpose, for example, to perform sensory reconstruction, treat nervous system disorders such as epilepsy and Parkinson, or implement other types of closed-loop control.

With the development of technologies, the electrode used by the stimulator is smaller and has higher density. However, a contact area between a high-density electrode of a smaller size and the biological tissue is significantly reduced, causing larger interface impedance, and therefore, the stimulator needs to apply a higher voltage to the electrode to stimulate the electrode to achieve specific effect. Because an application scenario is usually a biological tissue, and the stimulator is to be implanted in the biological tissue in a development trend, this voltage increase causes security challenges. Currently, there are no effective means to resolve a safety problem of the stimulator.

### SUMMARY

To resolve the foregoing problem, embodiments of this disclosure provide a method for controlling a stimulator, a stimulator, a brain-computer interface system, and a chip.

According to a first aspect of this disclosure, a method for controlling a stimulator is provided, and includes: obtaining a voltage of an electrode coupled to the stimulator; comparing the obtained electrode voltage with a first threshold voltage during a period when the stimulator does not apply an electrical pulse to the electrode; if magnitude (magnitude) of the electrode voltage is not less than magnitude of the first threshold voltage, generating a first control signal to control a drive circuit of the stimulator to apply at least one electrical pulse to the electrode, where a charge polarity of the at least one electrical pulse is opposite to a charge polarity indicated by the electrode voltage; and after applying the at least one electrical pulse, generating a second control signal to connect the electrode to a reference potential.

In the solution of this disclosure, residual charges are detected and active charge balancing and passive charge balancing are sequentially performed on excessively high residual charges, so that active charge balancing with high security may be used to offset a part of the residual charges, and the passive charge balancing may be used to quickly discharge the last remaining charges of a small quantity, to avoid an instantaneous large current in a balancing process and eliminate the residual charges with high precision, so as to ensure safety of a biological tissue and the electrode.

In an implementation of the first aspect, the first threshold voltage includes a first value associated with residual positive charges and a second value associated with residual negative charges. In this implementation, the residual charges of a positive polarity and the residual charges of a negative polarity can be effectively detected and eliminated.

In an implementation of the first aspect, the method further includes: generating a first stimulus signal to control the drive circuit to apply a first electrical pulse to the electrode, where the first electrical pulse has a first charge polarity; comparing the obtained electrode voltage with a second threshold voltage during the application of the first electrical pulse to the electrode; and if the magnitude of the electrode voltage is not less than magnitude of the second threshold voltage, disconnecting an electrical connection between a power supply of the drive circuit and the electrode and generating a third control signal to connect the electrode to the reference potential. In this implementation, short circuit protection can be implemented when the stimulator applies an electrical pulse, to prevent an excessively high short circuit voltage from being applied to the electrode and the biological tissue, so as to improve safety of the stimulator.

In an implementation of the first aspect, the method further includes: generating a second stimulus signal to control the drive circuit to apply a second electrical pulse to the electrode, where the second electrical pulse has a second charge polarity that is opposite to the first charge polarity; comparing the obtained electrode voltage with the second threshold voltage during the application of the second electrical pulse to the electrode; and if the magnitude of the electrode voltage is not less than the magnitude of the second threshold voltage, disconnecting the electrical connection between the power supply of the drive circuit and the electrode and generating the third control signal to connect the electrode to the reference potential. In this implementation, short circuit protection can be implemented when the stimulator applies an electrical pulse, to prevent an excessively high short circuit voltage from being applied to the electrode and the biological tissue, so as to improve the safety of the stimulator.

In an implementation of the first aspect, the second threshold voltage includes a third value associated with a short circuit protection positive voltage and a fourth value associated with a short circuit protection negative voltage. In this implementation, short circuit detection and protection for positive and negative power supply voltages can be implemented.

In an implementation of the first aspect, the second control signal is used to directly connect the electrode to the reference potential, and the third control signal is used to connect the electrode to the reference potential via a current limiting element. In this implementation, the electrode may be directly short-circuited to the reference potential in a passive charge balancing process of eliminating the residual charges to ensure a balancing speed and eliminate the remaining residual charges to a maximum extent, and the electrode is connected to the reference potential in a current limiting manner in a short circuit protection process to eliminate the residual charges and avoid an excessively large current that damages the biological tissue during a charge transfer process.

In an implementation of the first aspect, the method further includes: comparing the obtained electrode voltage with a third threshold voltage during a period between the application of the first electrical pulse and the application of the second electrical pulse; and if the magnitude of the electrode voltage is not less than magnitude of the third threshold voltage, generating an alert signal. In this implementation, whether a voltage change caused by single-time injected charges exceeds an electrochemical safety range can be effectively detected, to warn or alert an operator or a device in a timely manner to adjust an applied stimulus pulse when the injected charges are excessive.

In an implementation of the first aspect, the third threshold voltage includes a fifth value associated with an electrochemical-safe positive voltage and a sixth value associated with an electrochemical-safe negative voltage. In this implementation, electrochemical problems caused by positive charge injection and negative charge injection can be effectively detected and warned.

In an implementation of the first aspect, the obtaining a voltage of an electrode coupled to the stimulator includes: obtaining the electrode voltage at a predetermined frequency during a period when the stimulator does not apply the electrical pulse to the electrode. According to this embodiment, excessive charge accumulation can be detected in a timely manner, to ensure safety and avoid additional power consumption caused by real-time monitoring.

In an implementation of the first aspect, the generating a first control signal includes: determining at least one of a pulse width, a pulse amplitude, a pulse shape, and a pulse quantity of the at least one electrical pulse based on the electrode voltage; and generating the first control signal based on at least one of the determined pulse width, pulse amplitude, pulse shape, and pulse quantity. According to this embodiment, a pulse for the active balancing can be set based on a status of the residual charges, to eliminate the residual charges efficiently and accurately.

In an implementation of the first aspect, the generating a second control signal includes: determining duration for connecting the electrode to the reference potential based on the electrode voltage and the first control signal; and generating the second control signal based on the determined duration. In this implementation, setting of the passive charge balancing can be dynamically adjusted based on the residual charges and the active charge balancing, to improve effect and efficiency of the charge balancing.

According to a second aspect of this disclosure, a method for controlling a stimulator is provided, and the method includes: obtaining a voltage of an electrode coupled to the stimulator; generating a first stimulus signal to control a drive circuit of the stimulator to apply a first electrical pulse to the electrode, where the first electrical pulse has a first charge polarity; comparing the obtained electrode voltage with a second threshold voltage during the application of the first electrical pulse to the electrode; and if magnitude of the electrode voltage is not less than magnitude of the second threshold voltage, disconnecting an electrical connection between a power supply of the drive circuit and the electrode and generating a third control signal to connect the electrode to a reference potential.

In an implementation of the second aspect, the method further includes: generating a second stimulus signal to control the drive circuit to apply a second electrical pulse to the electrode, where the second electrical pulse has a second charge polarity that is opposite to the first charge polarity; comparing the obtained electrode voltage with the second threshold voltage during the application of the second electrical pulse to the electrode; and if the magnitude of the electrode voltage is not less than the magnitude of the second threshold voltage, disconnecting the electrical connection between the power supply of the drive circuit and the electrode and generating the third control signal to connect the electrode to the reference potential.

In an implementation of the second aspect, the second threshold voltage includes a third value associated with a short circuit protection positive voltage and a fourth value associated with a short circuit protection negative voltage.

In an implementation of the second aspect, the third control signal is used to connect the electrode to the reference potential via a current limiting element.

According to a third aspect of this disclosure, a method for controlling a stimulator is provided, and the method includes: obtaining a voltage of an electrode coupled to the stimulator; generating a first stimulus signal to control a drive circuit to apply a first electrical pulse to the electrode, where the first electrical pulse has a first charge polarity; generating a second stimulus signal to control the drive circuit to apply a second electrical pulse to the electrode, where the second electrical pulse has a second charge polarity that is opposite to the first charge polarity; comparing the obtained electrode voltage with a third threshold voltage during a period between the application of the first electrical pulse and the application of the second electrical pulse; and if magnitude of the electrode voltage is not less than magnitude of the third threshold voltage, generating an alert signal.

In an implementation of the third aspect, the third threshold voltage includes a fifth value associated with an electrochemical-safe positive voltage and a sixth value associated with an electrochemical-safe negative voltage.

According to a fourth aspect of this disclosure, a stimulator is provided, and the stimulator includes: a drive circuit, adapted to be coupled to an electrode; a detection circuit, adapted to be coupled to the electrode and configured to detect an electrode voltage; and a controller, coupled to the drive circuit and the detection circuit and configured to: obtain a voltage of the electrode; compare the obtained electrode voltage with a first threshold voltage during a period when the stimulator does not apply an electrical pulse to the electrode; if magnitude of the electrode voltage is not less than magnitude of the first threshold voltage, generate a first control signal to control the drive circuit to apply at least one electrical pulse to the electrode, where a charge polarity of the at least one electrical pulse is opposite to a charge polarity indicated by the electrode voltage; and after applying the at least one electrical pulse, generate a second control signal to connect the electrode to a reference potential.

In an implementation of the fourth aspect, the controller is further configured to: generate a first stimulus signal to control the drive circuit to apply a first electrical pulse to the electrode, where the first electrical pulse has a first charge polarity; comparing the obtained electrode voltage with a second threshold voltage during the application of the first electrical pulse to the electrode; and if the magnitude of the electrode voltage is not less than magnitude of the second threshold voltage, disconnect an electrical connection between a power supply of the drive circuit and the electrode and generate a third control signal to connect the electrode to the reference potential.

In an implementation of the fourth aspect, the controller is further configured to: generate a second stimulus signal to control the drive circuit to apply a second electrical pulse to the electrode, where the second electrical pulse has a second charge polarity that is opposite to the first charge polarity; compare the obtained electrode voltage with the second threshold voltage during the application of the second electrical pulse to the electrode; and if the magnitude of the electrode voltage is not less than the magnitude of the second threshold voltage, disconnect the electrical connection between the power supply of the drive circuit and the electrode and generate the third control signal to connect the electrode to the reference potential.

In an implementation of the fourth aspect, the controller is further configured to: compare the obtained electrode voltage with a third threshold voltage during a period between the application of the first electrical pulse and the application of the second electrical pulse; and if the magnitude of the electrode voltage is not less than magnitude of the third threshold voltage, generate an alert signal.

In an implementation of the fourth aspect, the controller includes a processing device and a comparison circuit, where the comparison circuit includes: a first multiplexer, configured to output one of a first value associated with residual positive charges, a third value associated with a short circuit protection positive voltage, and a fifth value associated with an electrochemical-safe positive voltage; a first comparator, configured to compare the electrode voltage detected by the detection circuit with a value output by the first multiplexer, and provide a comparison result for the processing device; a second multiplexer, configured to output one of a second value associated with residual negative charges, a fourth value associated with a short circuit protection negative voltage, and a sixth value associated with an electrochemical-safe negative voltage; and a second comparator, configured to compare the electrode voltage detected by the detection circuit with a value output by the second multiplexer and provide a comparison result for the processing device. In this implementation, comparison of a plurality of thresholds can be achieved in the stimulator in a simple, low-cost and efficient manner.

According to a fifth aspect of this disclosure, a stimulator is provided, and the stimulator includes: a drive circuit, adapted to be coupled to an electrode; a detection circuit, adapted to be coupled to the electrode and configured to detect an electrode voltage; and a controller, coupled to the drive circuit and the detection circuit and configured to: obtain a voltage of the electrode; generate a first stimulus signal to control the drive circuit to apply a first electrical pulse to the electrode, where the first electrical pulse has a first charge polarity; compare the obtained electrode voltage with a second threshold voltage during the application of the first electrical pulse to the electrode; and if magnitude of the electrode voltage is not less than magnitude of the second threshold voltage, disconnect an electrical connection between a power supply of the drive circuit and the electrode and generate a third control signal to connect the electrode to a reference potential.

In an implementation of the fifth aspect, the controller is further configured to: generate a second stimulus signal to control the drive circuit to apply a second electrical pulse to the electrode, where the second electrical pulse has a second charge polarity that is opposite to the first charge polarity; compare the obtained electrode voltage with the second threshold voltage during the application of the second electrical pulse to the electrode; and if the magnitude of the electrode voltage is not less than the magnitude of the second threshold voltage, disconnect the electrical connection between a power supply of the drive circuit and the electrode and generate a third control signal to connect the electrode to the reference potential.

In an implementation of the fifth aspect, the third control signal is used to connect the electrode to the reference potential via a current limiting element.

According to a sixth aspect of this disclosure, a stimulator is provided, and the stimulator includes: a drive circuit, adapted to be coupled to an electrode; a detection circuit, adapted to be coupled to the electrode and configured to detect an electrode voltage; and a controller, coupled to the drive circuit and the detection circuit and configured to: obtain a voltage of the electrode; generate a first stimulus signal to control the drive circuit to apply a first electrical pulse to the electrode, where the first electrical pulse has a first charge polarity; generate a second stimulus signal to control the drive circuit to apply a second electrical pulse to the electrode, where the second electrical pulse has a second charge polarity that is opposite to the first charge polarity; compare the obtained electrode voltage with a third threshold voltage during a period between the application of the first electrical pulse and the application of the second electrical pulse; and if magnitude of the electrode voltage is not less than magnitude of the third threshold voltage, generate an alert signal.

In an implementation of the sixth aspect, the stimulator further includes: a passive protection component, coupled between the drive circuit and the electrode, and resistance of the passive protection component increases as a current flowing through the passive protection component increases or a voltage crossing the passive protection component increases. In this implementation, when a short circuit occurs, the circuit can be quickly and effectively disconnected to prevent an excessively high voltage or large current from being applied to the electrode, to protect safety of a biological tissue and the electrode. In addition, use of passive protective components can also greatly reduce space or area occupation, to improve an integration degree of a component.

In an implementation of the sixth aspect, the drive circuit includes a first current source coupled between a power supply potential and the electrode and a second current source coupled between a ground potential and the electrode, and the passive protection component is coupled between the first current source and the electrode. In this implementation, the power supply potential can be prevented from being directly connected to the electrode when the first current source fails and is short-circuited.

In an implementation of the sixth aspect, the passive protection component includes a self-fuse component, and the self-fuse component is configured to fuse when the current flowing through the self-fuse component exceeds a fuse threshold.

In an implementation of the sixth aspect, the drive circuit includes a passive balancing branch, the passive balancing branch includes a first switch, a second switch, and a current limiting element, the first switch and a parallel circuit formed by the second switch and the current limiting element are connected in series between the electrode and the reference potential, resistance of the current limiting element is adjustable, and the controller is configured to control the first switch and the second switch to turn on and off. In this implementation, passive charge balancing of current limiting and non-current limiting can be effectively controlled and implemented.

In an implementation of the sixth aspect, the detection circuit includes a plurality of voltage divider resistors or a plurality of voltage divider capacitors that are connected in series. In this implementation, the electrode voltage can be accurately detected and the electrode voltage is attenuated to a voltage signal that can be processed in a low voltage domain.

According to a seventh aspect of this disclosure, a brain-computer interface system is provided, and includes an electrode and a stimulator in the fourth aspect, the fifth aspect, or the sixth aspect.

According to an eighth aspect of this disclosure, a chip is provided, and includes a processor, and a stimulator in the fourth aspect, the fifth aspect, or the sixth aspect.

In this application, based on the implementations provided in the foregoing aspects, the implementations may be further combined to provide more implementations. These aspects and other aspects of the present invention are simpler and easier to understand in descriptions of (a plurality of) embodiments below.

### BRIEF DESCRIPTION OF DRAWINGS

With reference to the accompanying drawings and the following detailed descriptions, the foregoing and other features, advantages, and aspects of embodiments of this disclosure become more apparent. In the accompanying drawings, same or similar reference numerals indicate same or similar elements.
FIG. 1 is a block diagram of a brain-computer interface system according to an embodiment of this disclosure;
FIG. 2 is a block diagram of a brain-computer interface system according to an embodiment of this disclosure;
FIG. 3 is a block diagram of a stimulator and an electrode according to an embodiment of this disclosure;
FIG. 4 is a diagram of a stimulator and a target object in a first phase according to an embodiment of this disclosure;
FIG. 5 is a diagram of a stimulator and a target object in a second phase according to an embodiment of this disclosure;
FIG. 6 is a diagram of a stimulator and a target object in a third phase according to an embodiment of this disclosure;
FIG. 7 is a schematic of an example circuit with a stimulator and an electrode according to an embodiment of this disclosure;
FIG. 8 is a waveform curve of an output voltage in a first example process of a stimulator according to an embodiment of this disclosure;
FIG. 9 is a waveform curve of an output voltage in a second example process of a stimulator according to an embodiment of this disclosure;
FIG. 10 is a waveform curve of an output voltage and an alert signal in a third example process of a stimulator according to an embodiment of this disclosure;
FIG. 11 is a schematic flowchart of a method for controlling a stimulator according to an embodiment of this disclosure;
FIG. 12 is a schematic flowchart of a method for controlling a stimulator according to an embodiment of this disclosure;
FIG. 13 is a schematic flowchart of a method for controlling a stimulator according to an embodiment of this disclosure; and
FIG. 14 is a schematic flowchart of a method for controlling a stimulator according to an embodiment of this disclosure.

### DESCRIPTION OF EMBODIMENTS

The following describes embodiments of this disclosure are in more detail with reference to the accompanying drawings. Although some embodiments of this disclosure are shown in the accompanying drawings, it should be understood that this disclosure can be implemented in various forms, and should not be construed as being limited to embodiments described herein, and instead, these embodiments are provided for a more thorough and complete understanding of this disclosure. It should be understood that the accompanying drawings and embodiments of this disclosure are merely used as examples and are not intended to limit the protection scope of this disclosure.

In the descriptions of embodiments of this disclosure, the term "including" and similar terms thereof shall be understood as non-exclusive inclusions, that is, "including but not limited to". The term "based on" should be understood as "at least partially based on". The term "one embodiment" or "this embodiment" should be understood as "at least one embodiment". The terms "first", "second", and the like may indicate different objects or a same object. Other explicit and implicit definitions may also be included below.

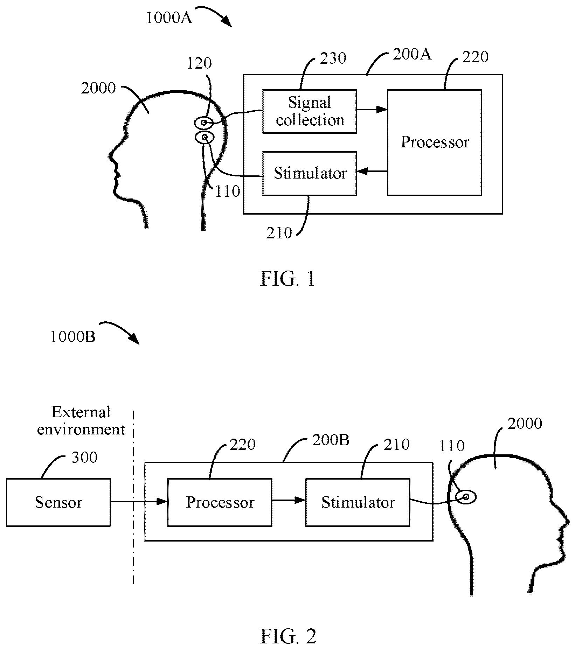

FIG. 1 is a block diagram of a brain-computer interface system 1000A according to an embodiment of this disclosure. The brain-computer interface system 1000A may be used for closed-loop control, disease treatment, or any other appropriate scenario. As shown in FIG. 1, the brain-computer interface system 1000A may include electrodes 110 and 120 and an interface chip 200A, and the interface chip 200A includes a stimulator 210, a processor 220, and a signal collector 230. The processor 220 may be, for example, an on-chip processing system. However, it may be understood that the stimulator 210, the processor 220, and the signal collector 230 may alternatively be disposed in another appropriate manner. For example, the stimulator 210, the processor 220, and the signal collector 230 may be disposed in chips that are separated from each other or an integrated circuit, or the processor 220 and the signal collector 230 are incorporated or integrated into the stimulator 210. This is not limited in this disclosure.

The signal collector 230 may obtain an electroencephalogram signal or a neural signal from a brain or a nervous system of a target object 2000 via the electrode 120, and send the collected signal to the processor 220. The processor 220 processes and analyzes the received signal. Then, the processor 220 may issue instructions to the stimulator 210 based on a result of the processing and analysis, and the stimulator 210 applies an expected stimulating pulse to a corresponding brain region or a corresponding neural region via the electrode 110. In merely an example, during application of the closed-loop control, the processor 220 may determine an action intention of the brain region of the brain by processing and analyzing the electroencephalogram signal. Then, the processor 220 may send instructions to the stimulator 210 based on the determined action intention, and the stimulator 210 applies, via the electrode 110, a stimulating pulse corresponding to the action intention to the corresponding brain region, to stimulate the target object 2000 to complete an action. In addition, in merely an example, during application of the disease treatment, the processor 220 determines, by processing and analyzing the electroencephalogram signal, whether the target object 2000 is currently in an illness state. If it is determined that the target object 2000 is in the illness state, the processor 220 sends instructions to the stimulator 210 to apply a stimulus pulse to the corresponding brain region via the electrode 110 based on a preset parameter, to intervene in occurrence of a disease.

FIG. 2 is a block diagram of a brain-computer interface system 1000B according to an embodiment of this disclosure. The brain-computer interface system 1000B may be used for open-loop control, sensory reconstruction, or other appropriate scenarios. As shown in FIG. 2, the brain-computer interface system 1000B may include an electrode 110, an interface chip 200B, and a sensor 300. The interface chip 200B includes a stimulator 210 and a processor 220. The processor 220 may be, for example, an on-chip processing system. It may be understood that the stimulator 210, the processor 220, and the sensor 300 may alternatively be disposed in another appropriate manner. For example, the sensor 300 may be disposed in the interface chip 200B, or the processor 220 and the sensor 300 may be disposed in chips that are separated from each other or an integrated circuit, or at least one of the processor 220 and the sensor 300 is incorporated or integrated into the stimulator 210. This is not limited in this disclosure.

The sensor 300 may collect environmental information from an external environment, convert the environmental information into an electrical signal, and provide the electrical signal for the processor 220. The processor 220 performs signal processing and conversion on the received electrical signal, to obtain stimulus information that can be responded to by a brain or a nervous system. Then, the stimulator 210 may apply stimulation to a corresponding area of the brain or the nervous system via the electrode 110 based on the stimulation information provided by the processor 220. In merely an example, during application of the sensory reconstruction, the sensor 220 may convert sensory information including vision, hearing, smell, taste, and touch in the external environment into the electrical signal. After the processor 220 processes and converts the electrical signal, the stimulator 210 applies a stimulus pulse to the corresponding brain region or neural region via the electrode 110, so that the target object 2000 generates or reconstructs the vision, the hearing, the smell, the taste, and the touch.

As described above, in some applications, such as the closed-loop control and the sensory reconstruction, the stimulator needs to use a higher-density electrode to obtain a high- precision control signal or reconstruct a high-resolution sensory signal. Compared with a previous electrode (for example, a millimeter-level electrode for the disease treatment), the high-density electrode is smaller in size, and may, for example, have a diameter less than 100 microns, and this causes a reduced contact area between the electrode and a biological tissue and increased interface impedance. Therefore, the stimulator needs to accordingly apply, to the electrode, a larger voltage that may be greater than 10V. An operation process of the stimulator faces some safety problems when the applied voltage is larger. These safety problems include biological tissue safety, electrode safety, and component failure safety. A current conventional scheme has some shortcomings in resolving the safety problem of the stimulator.

Embodiments of this disclosure provide an improved solution of a stimulator and for controlling the stimulator. In the improved solution, residual charges are detected and active charge balancing and passive charge balancing are performed in sequence when residual charges are excessively high, so that the residual charges on an electrode can be accurately eliminated to avoid damage to the electrode and a biological tissue caused by long-term charge accumulation, and damage to the biological tissue caused by an instantaneous high current during a charge balancing process can be avoided. In addition, in the improved solution, an electrode voltage at different operation phases of the stimulator may be monitored to determine whether a short circuit fault occurs and whether there is an electrochemical safety risk, to improve and ensure safety of the stimulator in a plurality of operation processes.

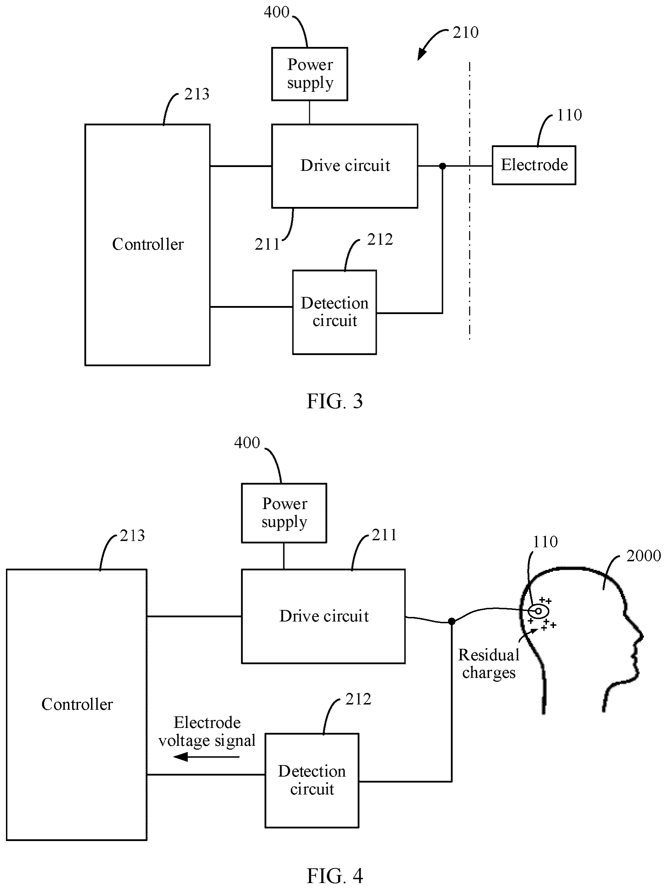

FIG. 3 is a block diagram of the stimulator 210 and the electrode 110 according to an embodiment of this disclosure. As shown in FIG. 3, the stimulator 210 may include a drive circuit 211, and the drive circuit 211 is coupled to the electrode 110. In an example, the electrode 110 may be attached to or implanted in a biological tissue, and the drive circuit 211 may be controlled to apply an electrical pulse of a positive charge polarity or a negative charge polarity to the electrode 110, so that the applied electrical pulse generates nerve stimulation in a brain or a nervous system. The drive circuit 211 can effectively increase a voltage of the electrical pulse, to generate a current pulse with a sufficiently high voltage to cope with a scenario in which electrode impedance is high. In an embodiment, the drive circuit 211 may be coupled to a power supply 400, and the power supply 400 may provide a high voltage for the drive circuit 211 to generate an electrical pulse sufficient to stimulate a nerve. Alternatively, the power supply 400 may be integrated into the drive circuit 211, and therefore, is a part of the drive circuit 211 instead of being disposed independently. The power supply 400 may include an energy storage apparatus such as a battery pack or a supercapacitor, a switch-mode power supply connected to a utility grid or the energy storage apparatus, any other type of power supply facility, or any combination thereof.

The drive circuit 211 may be further controlled to perform charge recovery on the electrode 110. Specifically, after the stimulator 210 applies an electrical pulse of a specific polarity to the biological tissue, the drive circuit 211 may continue to apply an electrical pulse of an opposite charge polarity to the biological tissue to perform charge recovery, to offset charges injected by the previous electrical pulse. For example, a quantity of charges of the latter pulse may be equal to a quantity of charges of the former pulse to ensure that a quantity of charges injected into the biological tissue is zero in a specific period.

In addition, the drive circuit 211 may be controlled to perform charge balancing on the electrode 110. Specifically, after the stimulator 210 stimulates the biological tissue, even if charge recovery is performed, there may still be excessive residual charges at an electrode interface and in the biological tissue due to various non-ideal effects. Long-term excessive charges damage the biological tissue and the electrode. Therefore, the drive circuit 211 may perform a charge balancing operation to eliminate the residual charges. More specifically, the drive circuit 211 may perform active charge balancing or passive charge balancing. To be specific, the drive circuit 211 may apply one or more small electrical pulses of a polarity that is opposite to a polarity of the residual charges to the electrode 110 to actively eliminate the residual charges, or the drive circuit 211 may connect the electrode 110 to an appropriate reference potential to passively discharge the residual charges.

The stimulator 210 includes a detection circuit 212, and the detection circuit 212 is coupled to the electrode 110. For example, the detection circuit 212 may be connected to a line between the drive circuit 211 and the electrode 110, or directly connected to the electrode 110, to detect a voltage of the electrode 110, and the detected voltage may indicate different status parameters in different operation phases. For example, the electrode voltage detected by the detection circuit 212 may indicate a status of the residual charges, or indicate whether a short circuit fault caused by a component failure exists, or indicate whether an electrochemical safety risk exists. In an embodiment, the detection circuit 212 may attenuate or decrease the voltage proportionally, so that the detected voltage signal can be provided for a low voltage domain for subsequent processing, and a component in the low voltage domain is not damaged by a signal with an excessively high voltage.

The stimulator 210 further includes a controller 213, and the controller 213 is coupled to the drive circuit 211 and the detection circuit 212. The controller 213 may be configured to control the drive circuit 211. In an embodiment, the controller 213 may be in the low voltage domain to process and analyze the detected signal, and the power supply 400 and the drive circuit 211 may be in a high voltage domain to provide a stimulus pulse with a sufficiently high voltage. Some or all control functions of the controller 213 may be implemented by using software or firmware, for example, implemented by using program code that can be executed by a computing apparatus, and the program code may be stored in a storage apparatus and executed by the computing apparatus, or implemented by using a hardware circuit such as a digital circuit or an analog circuit, or implemented by using a combination of software and a hardware circuit. Some examples of the controller 213 include but are not limited to a central processing unit (central processing unit, CPU), a digital signal processor (digital signal processor, DSP), a field programmable gate array (field programmable gate array, FPGA), an application-specific integrated circuit (application-specific integrated circuit, ASIC), an application-specific standard product (application-specific standard product, ASSP), a system on a chip (system on a chip, SoC), a complex programmable logic device (complex programmable logic device, CPLD), any appropriate processor or controller, and the like. In addition, although the controller 213 of the stimulator 210 shown in the figure and the processor 220 mentioned above are two separate control devices, the controller 213 and the processor 220 may alternatively be combined into one control device, and this may also implement embodiments of this disclosure.

FIG. 4 is a diagram of a stimulator 210 and a target object 2000 in a first phase according to an embodiment of this disclosure. In the first phase, a previous round of electrical pulse stimulation (which includes, for example, a first electrical pulse and a second electrical pulse described below) applied to the target object 2000 has been completed or has passed for a period of time, and the stimulator 210 has been reset and is waiting for a next round of electrical pulse stimulation. The following describes an operation of the stimulator 210 in the first phase with reference to FIG. 4.

In the first phase, the controller 213 of the stimulator 210 obtains the voltage of the electrode 110 from the detection circuit 212. In an example, the voltage of the electrode 110 indicates a status of residual charges at the electrode interface and in the biological tissue, and may also indicate an abnormal voltage caused by a short circuit. In some embodiments of this disclosure, the controller 213 may obtain the electrode voltage at a predetermined frequency during a period when the stimulator 210 does not apply an electrical pulse to the electrode 110. In this manner, in a low-risk scenario in which the stimulator 210 does not apply a stimulus electrical pulse, the status of the residual charges of the electrode 110 may be actively monitored periodically at a specific time interval, to discover excessive charge accumulation in a timely manner, so as to ensure safety and avoid additional power consumption caused by real-time monitoring.

The controller 213 compares the obtained electrode voltage with a first threshold voltage during a period when the stimulator 210 does not apply an electrical pulse to the electrode 110. For example, the controller 213 may preset or store the first threshold voltage. The first threshold voltage is related to a safety upper limit of the residual charges, and if magnitude of the electrode voltage exceeds magnitude of the first threshold voltage, it means that the residual charges are excessive and may cause damage to the biological tissue or the electrode. In some embodiments, the first threshold voltage may include a first value associated with residual positive charges and a second value associated with residual negative charges. In this case, the electrode voltage may be separately compared with the first value and the second value. Alternatively, the first threshold voltage may be an absolute value, and a comparison between the electrode voltage and the first threshold voltage may be a comparison between absolute values.

If the controller 213 determines that the magnitude of the electrode voltage is not less than the magnitude of the first threshold voltage, the controller 213 generates a first control signal to control the drive circuit 211 to apply at least one electrical pulse to the electrode 110, where a charge polarity of the at least one electrical pulse is opposite to a charge polarity indicated by the electrode voltage. For example, if the electrode voltage is not less than the first value associated with the residual positive charges or is not greater than the second value associated with the residual negative charges, or if the absolute value of the electrode voltage is not less than the first voltage threshold in a form of an absolute value, it means that the residual charges exceed a safety threshold. If the residual charges exceed the safety threshold and the charge polarity indicated by the electrode voltage is positive, the controller 213 may control the drive circuit 211 to send one or more electrical pulses of a negative charge polarity to the electrode 110, to offset the residual positive charges. If the residual charges exceed the safety threshold and the charge polarity indicated by the electrode voltage is negative, the controller 213 may control the drive circuit 211 to send one or more electrical pulses of a positive charge polarity to the electrode 110, to offset the residual negative charges. Therefore, the controller 213 may control the drive circuit 211 to perform active charge balancing on the residual charges. An electrical pulse used in this active charge balancing is usually a small pulse or a series of small pulses, and this can safely eliminate the residual charges step by step in a case of a large quantity of residual charges and avoid generating an instantaneous high current during a balancing process.

In some embodiments of this disclosure, the controller 213 may determine, based on the electrode voltage, at least one of a pulse width, a pulse amplitude, a pulse shape, and a pulse quantity of at least one electrical pulse to be applied. For example, the pulse width may be adjusted and determined based on the status that is of the residual charges and that is indicated by the electrode voltage, to offset the residual charges accurately. It may be understood that the pulse amplitude, the pulse shape, or the pulse quantity may also be adjusted based on the electrode voltage, or two, three, or all of the pulse width, the pulse amplitude, and the pulse quantity are adjusted based on the electrode voltage. In an embodiment, the controller 213 may further dynamically adjust at least one of the pulse width, the pulse amplitude, the pulse shape, and the pulse quantity of the electrical pulse in real time based on a change of the electrode voltage during a period when the at least one electrical pulse is applied. For example, after sending the one or more electrical pulses used to offset the residual charges, the controller 213 may dynamically adjust a pulse width of a subsequent electrical pulse based on a change status that is of residual charges and that is indicated by the electrode voltage. This helps actively apply the subsequent electrical pulse more accurately, and obtain a better charge balancing effect.

After applying the foregoing at least one electrical pulse, the controller 213 generates a second control signal to connect the electrode 110 to a reference potential. In an example, after the active charge balancing is performed, the residual charges at the electrode interface and in the biological tissue are greatly reduced, but there may still be a small quantity of charges. Therefore, the controller 213 may control the drive circuit 211 to connect the electrode 110 to a specific reference potential for passive charge balancing. The passive charge balancing may fix a potential of the electrode to the reference potential, to completely discharge the small quantity of remaining charges, and this effectively improves precision of charge balancing. In addition, because the passive charge balancing is performed after the active charge balancing, the passive charge balancing is used to discharge the small quantity of charges and does not generate an instantaneous large current that damages the biological tissue. In some embodiments of this disclosure, the controller 213 determines, based on the electrode voltage and the first control signal, duration for which the electrode 110 is connected to the reference potential, and generates the second control signal based on the determined duration. Specifically, the electrode voltage indicates the quantity of residual charges, and the controller 213 may estimate, based on the second control signal, a quantity of charges that the active charge balancing can be used to offset. Therefore, the controller 213 may estimate, through simple calculation, a quantity of charges that may still exist after the active charge balancing, and therefore, determine duration of the passive charge balancing, namely, duration required for discharging the remaining quantity of charges. In this manner, charge balancing can be completed with higher precision and efficiency, and a case in which there are still residual charges when passive balancing time is excessively short, or a case in which low efficiency is caused because passive balancing takes excessively much time is avoided.

It can be learned that the controller 213 coupled to the detection circuit 212 may control the drive circuit 211 based on the detected voltage provided by the detection circuit 212, to implement charge balancing. Therefore, when the charges at the electrode interface and in the biological tissue are excessive, timely processing and intervention may be performed to eliminate the excessive charges, and this effectively avoids damage to the biological tissue and the electrode due to stimulation and excessive residual charges during a long-term stimulation process. In addition, in some current conventional solutions, an instantaneous large current may be generated in a process of eliminating the residual charges, and the instantaneous large current has a disadvantageous effect on the biological tissue. In this embodiment of this disclosure, in the charge balancing process, the active charge balancing is performed before the passive charge balancing, so that a possibility of occurrence of the instantaneous large current in the charge balancing process is reduced or even avoided, and the residual charges may be completely eliminated with high precision, where for example, a quantity of the residual charges can be reduced to less than 0.1%.

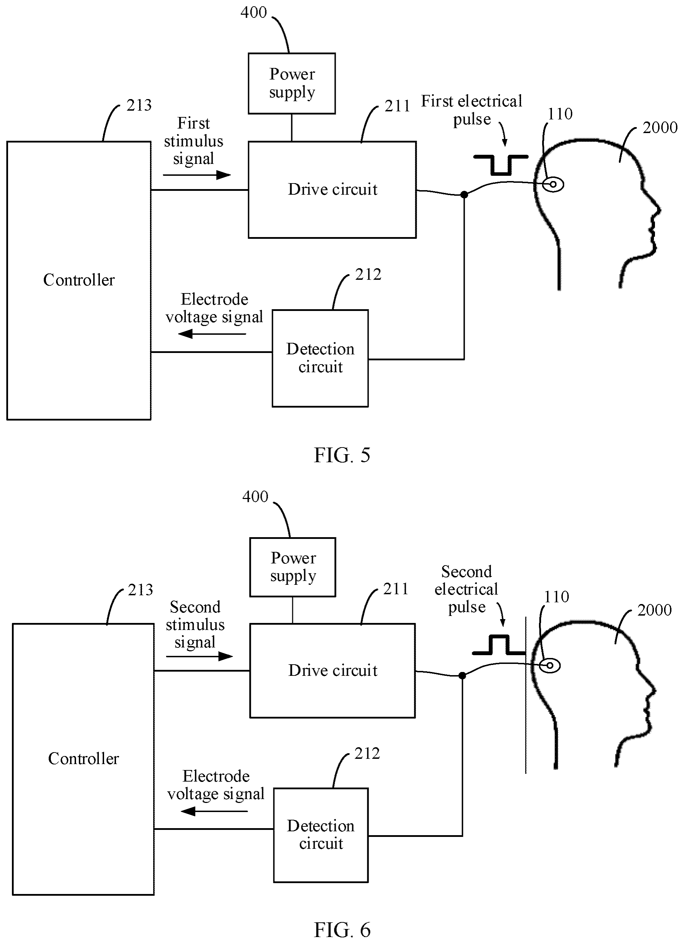

FIG. 5 is a diagram of a stimulator 210 and a target object 2000 in a second phase according to an embodiment of this disclosure. In the second phase, the stimulator 210 applies an electrical pulse to the target object 2000 to stimulate the brain or the nervous system. The following describes an operation of the stimulator 210 in the second phase with reference to FIG. 5.

In the second phase, the controller 213 obtains the voltage of the electrode 110 from the detection circuit 212. In an example, in a process of applying electrical pulse stimulation, if a short circuit fault occurs in the drive circuit 211, an entire power supply voltage or a part of the power supply voltage may be directly applied to the electrode 110, and this causes an excessively high electrode voltage. Therefore, by monitoring the electrode voltage, the controller 213 may determine whether a component failure or a short circuit fault occurs in the drive circuit 211 in a working process.

The controller 213 generates a first stimulus signal to control the drive circuit 211 to apply a first electrical pulse to the electrode 110, where the first electrical pulse has a first charge polarity. For example, the first charge polarity may be a negative charge polarity, and the drive circuit 211 amplifies the first stimulus signal and applies an electrical pulse of the negative charge polarity to the brain or the nervous system of the target object 2000, to perform neural stimulation on the target object 2000, so as to implement the foregoing objective of disease intervention, closed-loop control, or sensory reconstruction. However, it may be understood that the first charge polarity may alternatively be a positive charge polarity. This is not limited in this disclosure.

The controller 213 compares the obtained electrode voltage with a second threshold voltage during the application of the first electrical pulse to the electrode 110. For example, the controller 213 may preset or store the second threshold voltage. The first threshold voltage is related to a short circuit protection voltage. In addition, if magnitude of the electrode voltage exceeds magnitude of the second threshold voltage, it means that a short circuit fault or a component failure occurs in the drive circuit 211, and consequently, the part of or the entire power supply voltage is applied to the electrode 110. In some embodiments, the second threshold voltage may include a third value associated with a short circuit protection positive voltage and a fourth value associated with a short circuit protection negative voltage. In this case, the electrode voltage may be separately compared with the third value and the fourth value. Alternatively, the second threshold voltage may be an absolute value, and a comparison between the electrode voltage and the second threshold voltage may be a comparison between absolute values.

If the magnitude of the electrode voltage is not less than the magnitude of the second threshold voltage, the controller 213 disconnects an electrical connection between the power supply 400 of the drive circuit 211 and the electrode 110, and generates a third control signal to connect the electrode 110 to the reference potential. For example, if the electrode voltage is not less than the third value associated with the short circuit protection positive voltage or is not greater than the fourth value associated with the short circuit protection negative voltage, or if the absolute value of the electrode voltage is not less than the second voltage threshold in a form of an absolute value, it means that the magnitude of the electrode voltage exceeds a safety threshold. The electrode voltage exceeding the safety threshold means that a component failure or a short circuit fault may occur, and this may damage safety of the biological tissue. Therefore, the controller 213 may power off the power supply 400 of the drive circuit 211, or set the electrical connection between the power supply 400 and the electrode 110 to a high-resistance state, to prevent an excessively high voltage from being continuously applied to the electrode 110. In addition, the controller 213 further connects the electrode 110 to the reference potential, to discharge charges accumulated at the electrode 110 and around the electrode 110, so as to avoid damage caused by the accumulated charges to the biological tissue. In some embodiments of this disclosure, the third control signal is used to connect the electrode 110 to the reference potential in a current limiting manner. Specifically, when the electrode 110 is connected to the reference potential to discharge the accumulated charges, this passive charge balancing may cause an instantaneous large current if the accumulated charges are excessive. Therefore, the third control signal generated by the controller 213 may be used to discharge the charges in a current limiting manner, to limit current magnitude within a safe range.

FIG. 6 is a diagram of a stimulator 210 and a target object 2000 in a third phase according to an embodiment of this disclosure. In the second phase, the stimulator 210 applies an electrical pulse of an opposite charge polarity to the target object 2000 for charge recovery. The following describes an operation of the stimulator 210 in the third phase with reference to FIG. 6.

In the third phase, the controller 213 generates a second stimulus signal to control the drive circuit 211 to apply a second electrical pulse to the electrode 110, where the second electrical pulse has a second charge polarity that is opposite to the first charge polarity. For example, when the first charge polarity is negative, the second charge polarity may be a positive charge polarity, and the drive circuit 211 amplifies the second stimulus signal and applies the electrical pulse of the positive charge polarity to the brain or the nervous system of the target object 2000, to perform charge recovery on the electrical pulse of the negative charge polarity that is previously applied. In an embodiment, a quantity of charges applied by the second electrical pulse may be equal to a quantity of charges applied by the first electrical pulse, to ensure that a total quantity of charges applied by the two electrical pulses is zero.

The controller 213 compares the obtained electrode voltage with the second threshold voltage during the application of the second electrical pulse to the electrode 110. If the magnitude of the electrode voltage is not less than the magnitude the second threshold voltage, the controller 213 disconnects the electrical connection between the power supply 400 of the drive circuit 211 and the electrode 110, and generates the third control signal to connect the electrode 110 to the reference potential. Specifically, similar to the operation during the application of the first electrical pulse, if the controller 213 detects that the electrode voltage exceeds a threshold, it means that a short circuit fault or a component failure occurs in the drive circuit 211 during sending of the second electrical pulse. To prevent an excessively high voltage from being continuously applied to the electrode 110, the controller 213 may power off the power supply 400, or set the electrical connection between the power supply and the electrode 110 to a high-resistance state, and connect the electrode 110 to the reference potential to discharge the accumulated charges, to avoid damage to the biological tissue and the electrode and ensure safety of the target object 2000.

In some embodiments of this disclosure, during a period between the application of the first electrical pulse and the application of the second electrical pulse, the controller 213 may compare the obtained electrode voltage with a third threshold voltage. If the magnitude of the electrode voltage is not less than magnitude of the third threshold voltage, the controller 213 generates an alert signal. Specifically, the third threshold voltage is related to electrochemical safety. During an interval period after the second phase and before the third phase, the controller 213 may determine, by monitoring the electrode voltage, whether charges injected by applying the first electrical pulse during the second phase cause an excessively large voltage change that exceeds an electrochemical safety threshold. In general, a voltage change caused by single-time injected charges needs to be within a safe electrochemical window. It is usually not expected that the voltage exceeds the safe electrochemical window, which causes water electrolysis and electrochemical reactions and may have a disadvantageous effect on the biological tissue. However, in some cases, some stimulation operations may be required to inject appropriately excessive charges to make the voltage exceed the safe electrochemical window. Therefore, when the electrode voltage exceeds the electrochemical safety threshold, the controller 213 may only send the alert signal instead of directly intervening in. An operator or another device may perform an appropriate subsequent operation based on the fed-back alert signal, for example, reset a stimulus parameter or perform charge balancing in a timely manner, to avoid a continuous occurrence of the electrochemical reaction. Similar to the first threshold voltage and the second threshold voltage, in some embodiments, the third threshold voltage includes a fifth value associated with an electrochemical-safe positive voltage and a sixth value associated with an electrochemical-safe negative voltage. In this case, the electrode voltage may be separately compared with the fifth value and the sixth value. If the electrode voltage is not less than the fifth value associated with the electrochemical-safe positive voltage or is not greater than the sixth value associated with the electrochemical-safe negative voltage, it means that the single-time injected charges exceed the electrochemical safe range. Alternatively, the third threshold voltage may be an absolute value, and a comparison between the electrode voltage and the third threshold voltage may be a comparison between absolute values. During comparison, if the absolute value of the electrode voltage is not less than the second voltage threshold in a form of an absolute value, it means that the single-time injected charges exceed the electrochemical safety range.

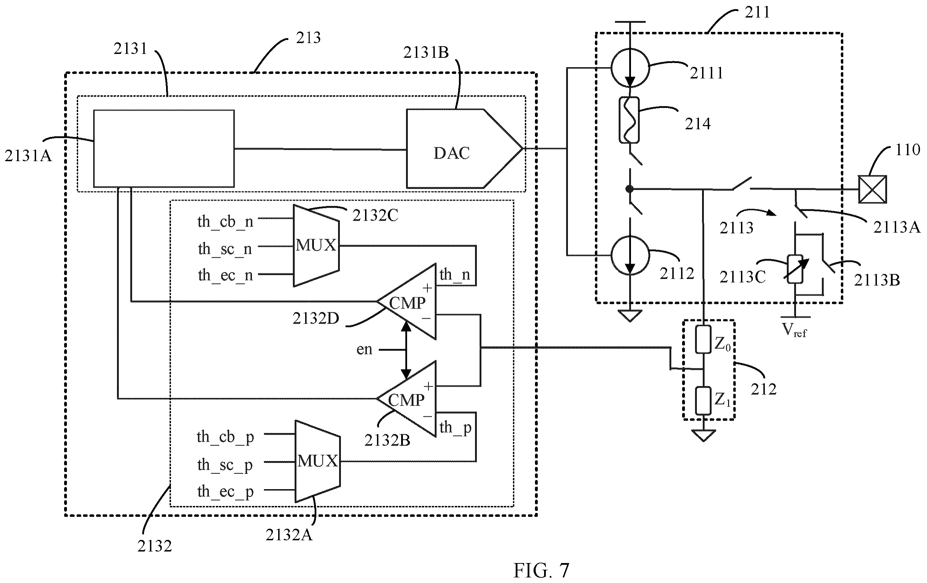

FIG. 7 is a schematic of an example circuit with a stimulator 210 and an electrode 110 according to an embodiment of this disclosure. As shown in FIG. 7, the controller 213 may include a processing device 2131 and a comparison circuit 2132. In an example, the processing device 2131 may include a processing unit 2131A and a digital-to-analog conversion unit 2131B. The processing unit 2131A may be configured to: generate the first stimulus signal and the second stimulus signal, and output pulse amplitude, width, and shape information to the digital-to-analog conversion unit 2131B based on a requirement to provide a specific pulse waveform. The digital-to-analog conversion unit 2131B converts the stimulus signal into an analog current pulse signal, and outputs the analog current pulse signal to the drive circuit 211. In addition, the processing unit 2131A also receives an output signal from the comparator circuit 2132 and performs threshold determining, to send the first control signal and the second control signal to perform active charge balancing and passive charge balancing, or to power off a power supply and perform passive charge balancing (for example, in a current limiting manner), or to send the alert signal to indicate that charge injection causes an electrochemical safety problem.

The comparator circuit 2132 includes a first multiplexer 2132A, a first comparator 2132B, a second multiplexer 2132C, and a second comparator 2132D. The first multiplexer 2132A and the first comparator 2132B are configured to perform safety threshold comparison when the residual charges are positive charges, a short circuit voltage may be a positive voltage, and the single-time injected charges are positive charges. The second multiplexer 2132C and the second comparator 2132D are configured to perform safety threshold comparison when the residual charges are negative charges, the short circuit voltage may be a negative voltage, and the single-time injected charges are negative charges.

The first multiplexer 2132A is configured to output one of the first value th_cb_p associated with the residual positive charges, the third value th_sc_p associated with the short circuit protection positive voltage, and the fifth value th_ec_p associated with the electrochemical-safe positive voltage. For example, the first multiplexer 2132A may select one of the first value th_cb_p, the third value th_sc_p, and the fifth value th_ec_p based on a selection enable signal at a selection terminal (not shown) of the first multiplexer 2132A, and the selection enable signal may be provided by the processing device 2132 or another appropriate device. For example, the first multiplexer 2132A outputs the first value th_cb_p in the first phase, outputs the third value th_sc_p in the second phase and the third phase, and outputs the fifth value th_ec_p in an interval period between the second phase and the third phase. The first comparator 2132B is configured to: compare the electrode voltage detected by the detection circuit 212 with a value th_p output by the first multiplexer 2132A, and provide a comparison result for the processing device 2131. The processing device 2131 may determine, based on the comparison result, whether to perform a charge balancing operation, a short circuit protection operation, or an electrochemical safety prompt.

The second multiplexer 2132C is configured to output one of the second value th_cb_n associated with the residual negative charges, the fourth value th_sc_n associated with the short circuit protection negative voltage, and the sixth value th_ec_n associated with the electrochemical-safe negative voltage. Similar to the first multiplexer 2132A, the second multiplexer 2132C may output the second value th_cb_n, the fourth value th_sc_n, or the sixth value th_ec_n based on the selection enable signal. For example, the second multiplexer 2132C outputs the second value th_cb_n in the first phase, outputs the fourth value th_sc_n in the second phase and the third phase, and outputs the sixth value th_ec_n in the interval period between the second phase and the third phase. The second comparator 2132D is configured to: compare the electrode voltage detected by the detection circuit 212 with a value th_n output by the second multiplexer 2132C and provide a comparison result for the processing device 2131. The processing device 2131 may determine, based on the comparison result, whether to perform the charge balancing operation, the short circuit protection operation, or the electrochemical safety prompt.

It may be understood that the controller 213 in FIG. 7 is merely an example rather than a limitation, and the controller 213 may be implemented in any other appropriate manner. For example, a function of the comparison circuit 2132 may alternatively be implemented in a manner of software or firmware and implemented by the processing device 2131, or the digital-to-analog conversion unit 2131B may be integrated into the processing unit 2131A and does not need to be disposed separately.

The drive circuit 211 may include a first current source 2111 coupled between a power supply potential and the electrode 110 and a second current source 2112 coupled between a ground potential and the electrode 110. Specifically, the first current source 2111 and the second current source 2112 may generate, under control of the processing device 2131, corresponding electrical pulses to be applied to the electrode 110. For example, the first current source 2111 generates a current pulse of a positive charge polarity based on the first stimulus signal from the processing device 2131, and the second current source 2112 generates a current pulse of a negative charge polarity based on the second stimulus signal from the processing device 2131. In an embodiment, switches may be further separately disposed on branches on which the first current source 2111 and the second current source 2112 are located, to turn off, when one current source generates a current pulse, a branch in which the other current source is located.

The drive circuit 211 may further include a passive balancing branch 2113, and the passive balancing branch 2113 includes a first switch 2113A, a second switch 2113B, and a current limiting element 2113C. The first switch 2113A and a parallel circuit formed by the second switch 2113B and the current limiting element 2113C are connected in series between the electrode 110 and the reference potential V_{ref}, and the controller 213 is configured to control the first switch 2113A and the second switch 2113B to turn on and off. For example, in the first phase, when passive charge balancing is performed after active charge balancing, the controller 213 may turn on both the first switch 2113A and the second switch 2113B, to directly connect the electrode 110 to the reference potential V_{ref}, so as to quickly discharge a small quantity of remaining charges in short time. In addition, because the quantity of charges is small, there is no instantaneous large current. In the second phase and the third phase, when a short circuit occurs, the controller 213 may turn on the first switch 2113A and turn off the second switch 2113B after powering off the power supply, so that the electrode 110 is connected to the reference potential V_{ref} via the current limiting element 2113C. This effectively limits a current in the passive balancing, to avoid damage to the biological tissue caused by an excessively large current. In an embodiment, resistance of the current limiting element 2113C is adjustable, for example, may be adjusted by the controller 213 or another device. In an example, the current limiting element 2113C may be a variable resistor. Therefore, the resistance of the current limiting element 2113C may be adjusted based on charges that need to be discharged. For example, the resistance of the current limiting element 2113C may be increased when a quantity of charges is excessively large, to avoid an instantaneous large current, or the resistance of the current limiting element 2113C may be decreased when a quantity of charges is small, to avoid low efficiency caused by an excessively slow speed of discharging the charges.

It may be understood that an implementation of the drive circuit 211 shown in FIG. 7 is merely an example rather than a limitation, and the drive circuit 211 may be implemented in any other appropriate manner.

The detection circuit 212 may include a plurality of voltage divider resistors Z₀ and Z₁ that are connected in series. Specifically, the controller 213 may be in a low voltage domain, and the power supply 400 and the drive circuit 211 may be in a high voltage domain. Therefore, when an electrical pulse is applied, an electrode voltage in the high voltage domain is relatively high. Two or more voltage divider resistors are used, so that the electrode voltage may be attenuated or reduced to a low voltage proportionally, to facilitate analysis and processing of a detection signal in the low voltage domain, and prevent a high voltage signal from damaging a component in the low voltage domain. The controller 213 is placed in the low voltage domain and the drive circuit 211 is placed in the high voltage domain, so that signal processing and analysis may be performed with a low voltage and low power consumption, and a stimulus pulse with a sufficiently large voltage may be provided. In an alternative embodiment, the detection circuit 212 may include a plurality of voltage divider capacitors that are connected in series. The capacitors are used for voltage division, so that a voltage attenuation effect similar to that of using resistors for voltage division can be implemented, and a direct current component can be effectively isolated, to protect a component in the low voltage domain. In an implementation in which the plurality of voltage divider capacitors are used in the detection circuit 212, the detection circuit 212 needs to cooperate with the passive balancing branch 2113. For example, before the stimulator 210 is used, the passive balancing branch 2113 is used to discharge residual charges in the voltage divider capacitors in advance, to avoid affecting a subsequent operation. It may be understood that an implementation of the detection circuit 212 shown in FIG. 7 is merely an example rather than a limitation, and the detection circuit 212 may be implemented in any other appropriate manner.

In some embodiments of this disclosure, the stimulator 210 may further include a passive protection component 214, and the passive protection component 214 is coupled between the drive circuit 211 and the electrode 110. Resistance of the passive protection component 214 increases as a current flowing through the passive protection component 214 increases or a voltage crossing the passive protection component 214 increases. In this manner, when a large current or a high voltage occurs due to a short circuit, the passive protection component 214 may change to a high impedance state, to prevent the excessively high voltage or large current from being applied to the electrode 211, so as to protect safety of the biological tissue and the electrode. In an embodiment, the passive protection component 214 may be a self-fuse component, for example, an electronic fuse, and may fuse when a current flowing through the passive protection component 214 exceeds a fuse threshold, to implement a protection function. In an embodiment, the passive protection component 214 may be coupled between the first current source 2111 and the electrode 110. For example, when the stimulator 210 operates normally, resistance of the passive protection component 214 is very low, and therefore, output of the stimulus electrical pulse is not affected. If the first current source 2111 coupled to the power supply potential is short-circuited due to a failure, the passive protection component 214 may change a circuit between the power supply potential and the electrode 110 to a high impedance state or disconnect the circuit, to prevent the power supply potential from being directly connected to the electrode 110.

Use of the passive protective component like the self-fuse component in the stimulator 210 has definite benefits. Specifically, in some conventional solutions, a coupling capacitor is inserted between the stimulator and the electrode. Therefore, even if a short circuit occurs, a high voltage that has a safety risk is not applied to the electrode and the biological tissue provided that the coupling capacitor is not broken down. However, to ensure that most of charges output by the stimulator are injected into the biological tissue via the electrode, instead of being lost on the coupling capacitor, a capacitance value of the coupling capacitor needs to be far greater than an equivalent capacitance in an electrode model. Considering that the equivalent capacitance in the electrode model is usually more than dozens of nF, the capacitance value of the coupling capacitor is usually in a unit of µF. This coupling capacitor cannot be integrated on a chip because the coupling capacitor is excessively large. In comparison, an occupied area may be greatly reduced by using the passive protection component like the self-fuse component. For example, an occupied area of each of some self-fuse components is only 1/25000 of that of the coupling capacitor, to effectively reduce space and area occupation and improve an integration degree of the component.

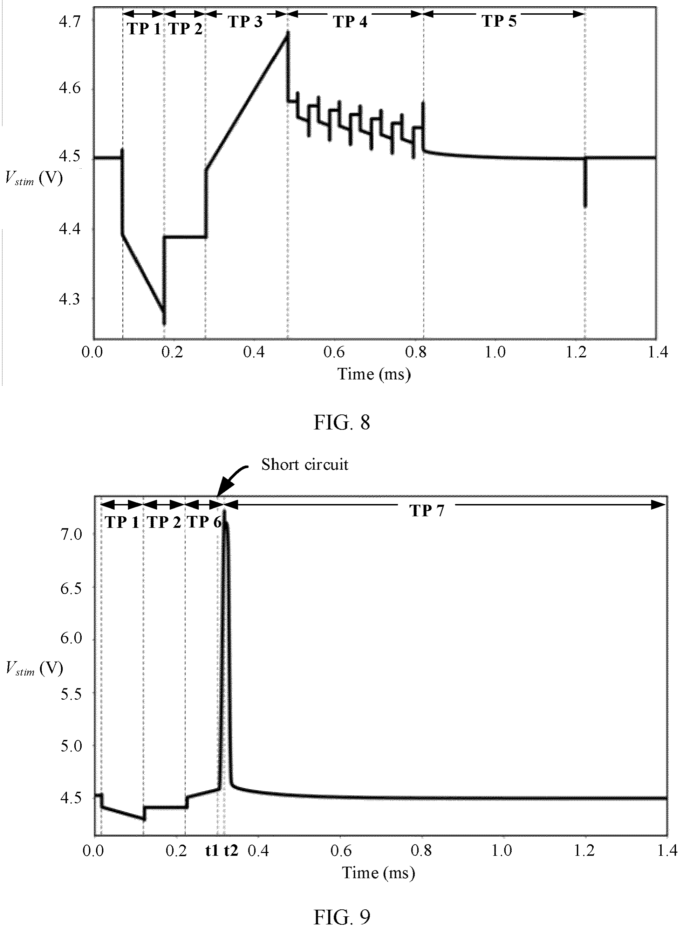

FIG. 8 is a waveform curve of an output voltage in a first example process of a stimulator 210 according to an embodiment of this disclosure. As shown in FIG. 8, during a TP 1 period, the first stimulus signal generated by the controller 213 outputs a constant negative current pulse as the first electrical pulse to the electrode 110 via the drive circuit 211. Due to a capacitance characteristic of the electrode 110, the electrode voltage decreases gradually over time. During this period, the stimulator 210 does not perform any short circuit protection operation because no short circuit occurs. During a TP 2 period, the first electrical pulse ends, and in this case, the voltage on the electrode 110 is caused by charges injected by the first electrical pulse. Because a voltage change is within the electrochemical safety range, the controller 213 of the stimulator 210 does not generate any alert signal. During a TP 3 period, the second stimulus signal generated by the controller 213 outputs a constant positive current pulse as the second electrical pulse to the electrode 110 via the drive circuit 211. Similarly, due to the capacitance characteristic of the electrode, the electrode voltage gradually increases over time, and the stimulator 210 does not perform any short circuit protection operation because no short circuit occurs. During TP 4 and TP 5 periods, the second electrical pulse ends, and the stimulator 210 performs a charge balancing operation to eliminate residual charges. The stimulator 210 first performs active charge balancing during the TP 4 period. Because the residual charges after the second electrical pulse ends are positive charges and exceed the safety threshold, the controller 213 controls the drive circuit 211 to output a series of electrical pulses of a negative charge polarity with a small amplitude and a small pulse width to the electrode 110, until a quantity of the residual charges is gradually reduced to a value less than a specific threshold. Then, the stimulator 210 performs passive charge balancing during the TP 5 period, and after a period of time, the potential of the electrode 110 decreases to the reference potential.

FIG. 9 is a waveform curve of an output voltage in a second example process of a stimulator 210 according to an embodiment of this disclosure. Similar to the first example process of FIG. 8, in FIG. 9, the stimulator 210 outputs the first electrical pulse with a negative charge polarity to the electrode 110 during a TP 1 period and performs electrochemical safety detection during a TP 2 period. During the TP 1 period and the TP 2 period, no short circuit fault or electrochemical safety problem occurs. During a TP 6 period, the stimulator 210 outputs the second electrical pulse with a positive charge polarity to the electrode 110. At a moment t1 during the TP 6 period, a short circuit fault caused by a component failure occurs in the stimulator 210, where for example, the first current source 2111 is short-circuited. Therefore, a large quantity of charges are injected into the biological tissue via the electrode 110, causing a rapid rise of the electrode voltage. At a moment t2 at an end of the TP 6 period, the controller 213 finds, via the detection circuit 212, that the electrode voltage exceeds the safety threshold, and performs a short circuit protection operation. In this way, the electrical connection between the power supply and the electrode 110 is disconnected. For example, the controller 213 actively controls a switch on a branch on which the first current source 2111 is located to be turned off, to power off the power supply, and/or cause the passive protection component 214 to be set to a high impedance state or fused. During a T7 period, the controller 213 connects the electrode 110 to the reference potential via the current limiting element 2113C for charge balancing. Within time of dozens of microseconds, the voltage of the electrode 110 is restored to a normal voltage, to avoid long-time accumulation of a large quantity of charges in the biological tissue.

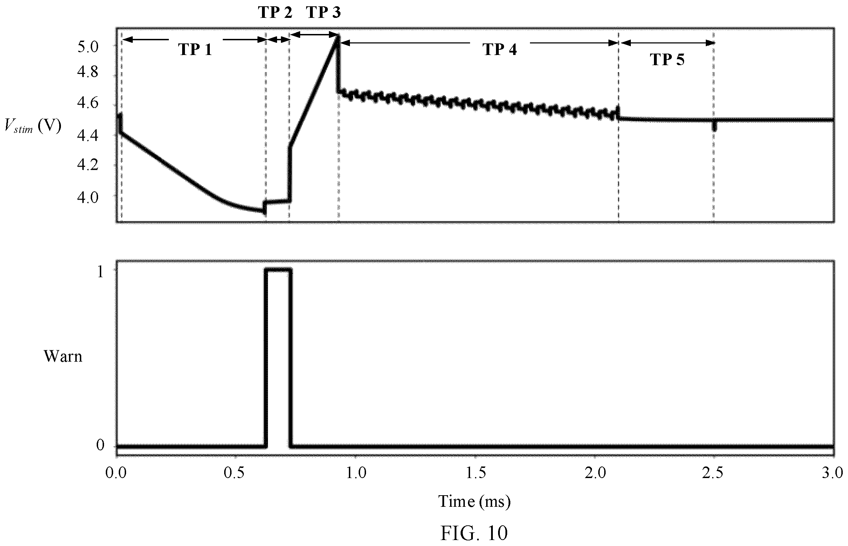

FIG. 10 is a waveform curve of an output voltage and an alert signal in a third example process of a stimulator 210 according to an embodiment of this disclosure. Similar to the first example process of FIG. 8, in FIG. 10, the stimulator 210 applies the first electrical pulse to the electrode 110 during a TP 1 period, performs electrochemical safety detection during a TP 2 period, applies the second electrical pulse to the electrode 110 during a TP 3 period, and performs active charge balancing and passive charge balancing during TP 4 and TP 5 periods respectively. A difference from the first example process is that during the TP 1 period, the stimulator 210 injects excessive charges into the biological tissue during the application of the first electrical pulse, causing an excessively large voltage change at the electrode interface. During the TP 2 period, the controller 213 of the stimulator 210 finds that the voltage exceeds the electrochemical safety range and therefore, generates a high level pulse signal as the alert signal to alert or warn the operator or the another device.

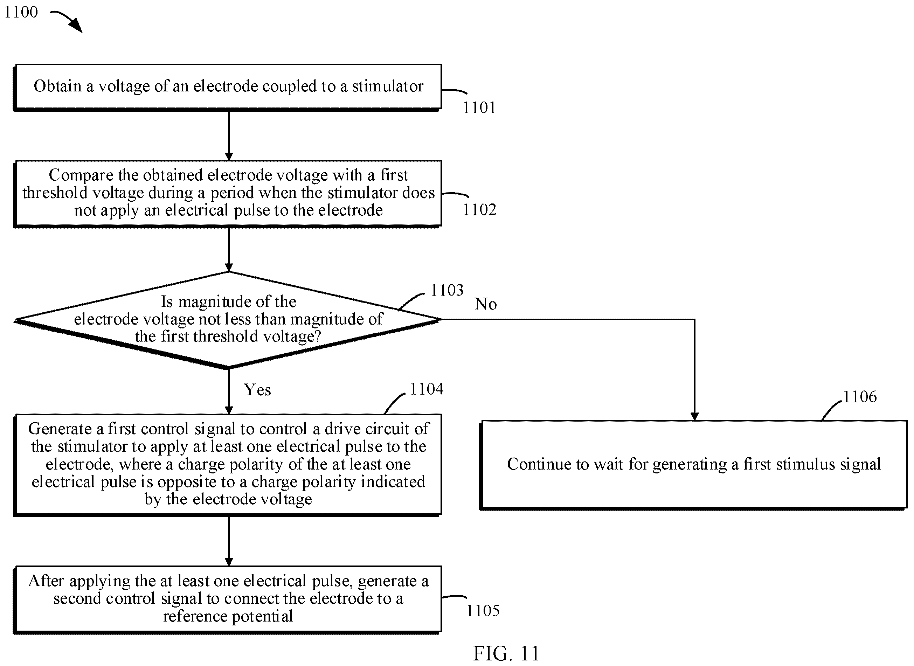

With reference to FIG. 1 to FIG. 10, the foregoing describes in detail the stimulator 210 and the brain-computer interface systems 1000A and 1000B including the stimulator 210 provided in this application. With reference to FIG. 11 to FIG. 14, the following describes a control method provided based on the stimulator 210 according to this application.

FIG. 11 is a schematic flowchart of a method 1100 for controlling a stimulator 210 according to an embodiment of this disclosure. The method 1100 may be implemented in the stimulator 210 of FIG. 1 to FIG. 3 and FIG. 7, and may be performed, for example, by a controller 213 of the stimulator 210. It may be understood that the foregoing aspects described in FIG. 1 to FIG. 3 and FIG. 7 is applicable to the method 1100. For purposes of discussion, the method 1100 is described with reference to FIG. 1 to FIG. 3 and FIG. 7.

At a block 1101, the controller 213 obtains a voltage of an electrode 110 coupled to the stimulator 210. In some embodiments, the controller 213 obtains the electrode voltage at a predetermined frequency during a period when the stimulator 210 does not apply an electrical pulse to the electrode 110.

At a block 1102, the controller 213 compares the obtained electrode voltage with a first threshold voltage during the period when the stimulator 210 does not apply an electrical pulse to the electrode 110. In some embodiments, the first threshold voltage includes a first value th_cb_p associated with residual positive charges and a second value th_cb_n associated with residual negative charges.

At a block 1103, the controller 213 determines whether magnitude of the electrode voltage is not less than magnitude of the first threshold voltage.

At a block 1104, if the magnitude of the electrode voltage is not less than the magnitude of the first threshold voltage, the controller 213 generates a first control signal to control a drive circuit 211 of the stimulator 210 to apply at least one electrical pulse to the electrode 110, where a charge polarity of the at least one electrical pulse is opposite to a charge polarity indicated by the electrode voltage. In some embodiments, the controller 213 determines at least one of a pulse width, a pulse amplitude, a pulse shape, and a pulse quantity of the at least one electrical pulse based on the electrode voltage, and generates the first control signal based on at least one of the determined pulse width, pulse amplitude, pulse shape, and pulse quantity.

At a block 1105, after applying the at least one electrical pulse, the controller 213 generates a second control signal to connect the electrode 110 to a reference potential V_{ref}. In some embodiments, the second control signal is used to directly connect the electrode 110 to the reference potential V_{ref}. In some embodiments, the controller 213 determines, based on the electrode voltage and the first control signal, duration for which the electrode 110 is connected to the reference potential V_{ref}, and generates the second control signal based on the determined duration.

At a block 1106, if the magnitude of the electrode voltage is less than the magnitude of the first threshold voltage, the controller 213 continues to wait for generating a first stimulus signal.

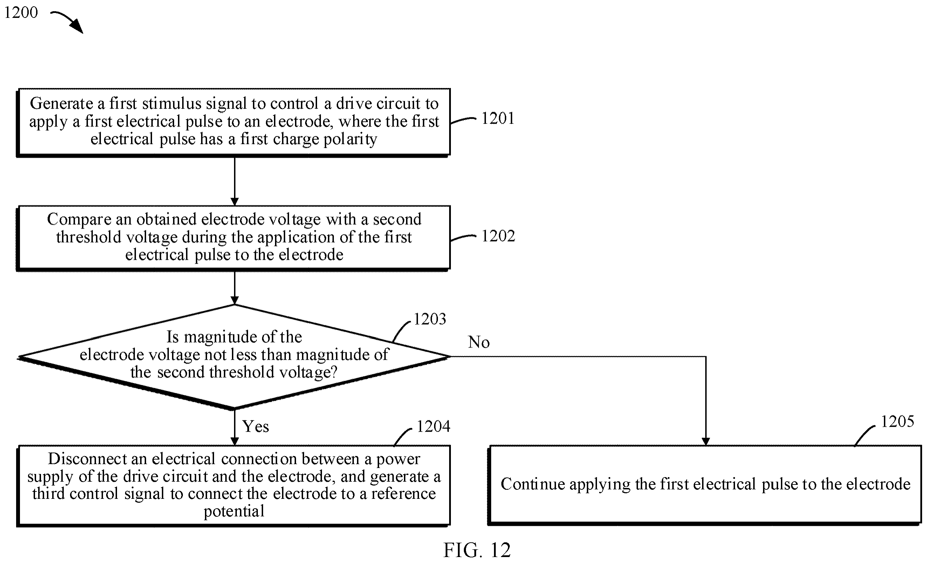

FIG. 12 is a schematic flowchart of a method 1200 for controlling a stimulator 210 according to an embodiment of this disclosure. The method 1200 may be implemented in the stimulator 210 of FIG. 1 to FIG. 3 and FIG. 7, and may be performed, for example, by a controller 213 of the stimulator 210. The method 1200 may be performed after or before the method 1100, or may be performed independently.

At a block 1201, the controller 213 generates a first stimulus signal to control a drive circuit 211 of the stimulator 210 to apply a first electrical pulse to the electrode 110, where the first electrical pulse has a first charge polarity.

At a block 1202, the controller 213 compares an obtained electrode voltage with a second threshold voltage during the application of the first electrical pulse to the electrode 110. In some embodiments, the second threshold voltage includes a third value th_sc_p associated with a short circuit protection positive voltage and a fourth value th_sc_n associated with a short circuit protection negative voltage.

At a block 1203, the controller 213 determines whether magnitude of the electrode voltage is not less than magnitude of the second threshold voltage.

At a block 1204, if the magnitude of the electrode voltage is not less than the magnitude of the second threshold voltage, the electrical connection is disconnected between a power supply 400 of the drive circuit 211 and the electrode 110, and a third control signal is generated to connect the electrode 110 to a reference potential V_{ref}. In some embodiments, the third control signal is used to connect the electrode 110 to the reference potential V_{ref} via a current limiting element 2113C.

At a block 1205, if the magnitude of the electrode voltage is less than the magnitude of the second threshold voltage, the controller 213 controls the drive circuit 211 to continue applying the first electrical pulse to the electrode 110.

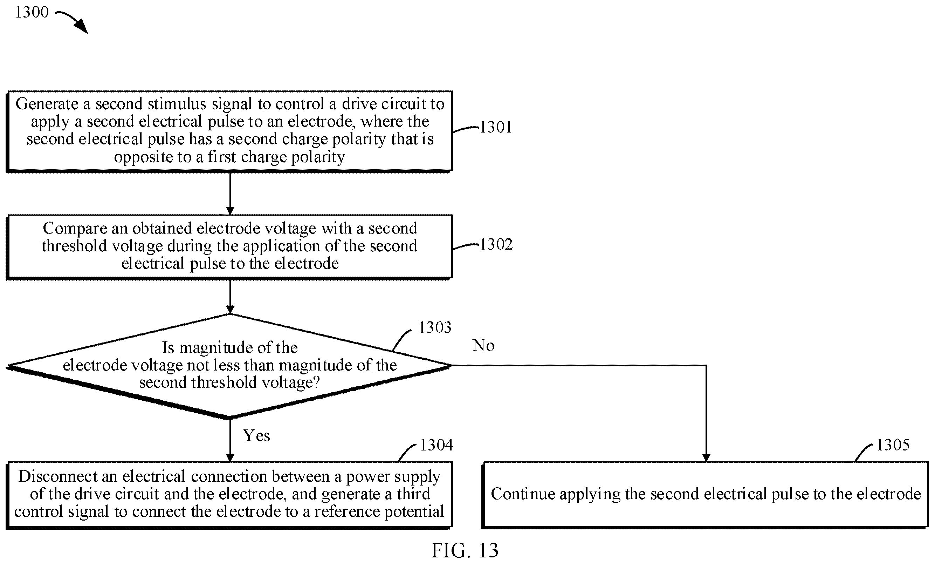

FIG. 13 is a schematic flowchart of a method 1300 for controlling a stimulator 210 according to an embodiment of this disclosure. The method 1300 may be implemented in the stimulator 210 of FIG. 1 to FIG. 3 and FIG. 7, and may be performed, for example, by a controller 213 of the stimulator 210. The method 1300 may be performed after the method 1200.

At a block 1301, the controller 213 generates a second stimulus signal to control the drive circuit 211 to apply a second electrical pulse to an electrode 110, where the second electrical pulse has a second charge polarity that is opposite to a first charge polarity.

At a block 1302, the controller 213 compares an obtained electrode voltage with a second threshold voltage during the application of the second electrical pulse to the electrode 110.

At a block 1303, the controller 213 determines whether magnitude of the electrode voltage is not less than magnitude of the second threshold voltage.

At a block 1304, if the magnitude of the electrode voltage is not less than the magnitude of the second threshold voltage, the controller 213 disconnects an electrical connection between a power supply 400 of the drive circuit 211 and the electrode 110, and generates a third control signal to connect the electrode 110 to a reference potential V_{ref}.

At a block 1305, if the magnitude of the electrode voltage is less than the magnitude of the second threshold voltage, the controller 213 controls the drive circuit 211 to continue applying the second electrical pulse to the electrode 110.

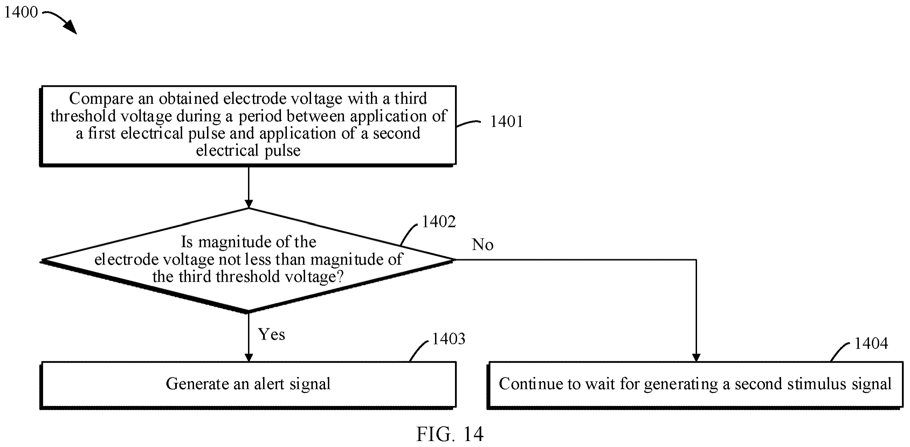

FIG. 14 is a schematic flowchart of a method 1400 for controlling a stimulator 210 according to an embodiment of this disclosure. The method 1400 may be implemented in the stimulator 210 of FIG. 1 to FIG. 3 and FIG. 7, and may be performed, for example, by a controller 213 of the stimulator 210. The method 1400 may be performed after the method 1100 and before the method 1200.

At a block 1401, during a period between application of a first electrical pulse and application of a second electrical pulse, the controller 213 compares an obtained electrode voltage with a third threshold voltage. In some embodiments, the third threshold voltage includes a fifth value th_ec_p associated with an electrochemical-safe positive voltage and a sixth value th_ec_n associated with an electrochemical-safe negative voltage.

At a block 1402, the controller 213 determines whether magnitude of the electrode voltage is not less than magnitude of the third threshold voltage.

At a block 1403, if the magnitude of the electrode voltage is not less than the magnitude of the third threshold voltage, the controller 213 generates an alert signal.

At a block 1404, if the magnitude of the electrode voltage is less than the magnitude of the third threshold voltage, the controller 213 continues to wait for generating a second stimulus signal.

From the teachings given in the foregoing descriptions and the related accompanying drawings, many of the modified forms and other implementations of this disclosure given herein will be realized by a person skilled in the art related to this disclosure. Therefore, it is to be understood that the implementations of this disclosure are not limited to the disclosed specific implementations, and modifications and other implementations are intended to fall within the scope of this disclosure. Further, although the foregoing description and related drawings describe example implementations in the context of some example combinations of parts and/or functions, it should be appreciated that different combinations of parts and/or functions may be provided by alternative implementations without departing from the scope of this disclosure. In this regard, for example, other combinations of components and/or functions that are different from those explicitly described above are also expected to fall within the scope of this disclosure. Although specific terms are used herein, the specific terms are used only in general and descriptive meanings and are not intended to be limited.

## Claims

1. A method for controlling a stimulator (210), comprising:
obtaining a voltage of an electrode (110) coupled to the stimulator (210);
comparing the obtained electrode voltage with a first threshold voltage during a period when the stimulator (210) does not apply an electrical pulse to the electrode (110);
if magnitude of the electrode voltage is not less than magnitude of the first threshold voltage, generating a first control signal to control a drive circuit (211) of the stimulator (210) to apply at least one electrical pulse to the electrode (110), wherein a charge polarity of the at least one electrical pulse is opposite to a charge polarity indicated by the electrode voltage; and
after applying the at least one electrical pulse, generating a second control signal to connect the electrode (110) to a reference potential (V_{ref}).

2. The method according to claim 1, further comprising:
generating a first stimulus signal to control the drive circuit (211) to apply a first electrical pulse to the electrode (110), wherein the first electrical pulse has a first charge polarity;
comparing the obtained electrode voltage with a second threshold voltage during the application of the first electrical pulse to the electrode (110); and
if the magnitude of the electrode voltage is not less than magnitude of the second threshold voltage, disconnecting an electrical connection between a power supply (400) of the drive circuit (211) and the electrode (110) and generating a third control signal to connect the electrode (110) to the reference potential (V_{ref}).

3. The method according to claim 2, further comprising:
generating a second stimulus signal to control the drive circuit (211) to apply a second electrical pulse to the electrode (110), wherein the second electrical pulse has a second charge polarity that is opposite to the first charge polarity;
comparing the obtained electrode voltage with the second threshold voltage during the application of the second electrical pulse to the electrode (110); and
if the magnitude of the electrode voltage is not less than the magnitude of the second threshold voltage, disconnecting the electrical connection between the power supply (400) of the drive circuit (211) and the electrode (110) and generating the third control signal to connect the electrode (110) to the reference potential (V_{ref}).

4. The method according to claim 2 or 3, wherein the second control signal is used to directly connect the electrode (110) to the reference potential (V_{ref}), and the third control signal is used to connect the electrode (110) to the reference potential (V_{ref}) via a current limiting element (2113C).

5. The method according to claim 3, further comprising:
comparing the obtained electrode voltage with a third threshold voltage during a period between the application of the first electrical pulse and the application of the second electrical pulse; and
if the magnitude of the electrode voltage is not less than magnitude of the third threshold voltage, generating an alert signal.

6. The method according to claim 1, wherein the obtaining a voltage of an electrode (110) coupled to the stimulator (210) comprises:
obtaining the electrode voltage at a predetermined frequency during a period when the stimulator (210) does not apply the electrical pulse to the electrode (110).

7. The method according to claim 1, wherein the generating a first control signal comprises:
determining at least one of a pulse width, a pulse amplitude, a pulse shape, and a pulse quantity of the at least one electrical pulse based on the electrode voltage; and
generating the first control signal based on at least one of the determined pulse width, pulse amplitude, pulse shape, and pulse quantity.

8. The method according to claim 1, wherein the generating a second control signal comprises:
determining duration for connecting the electrode (110) to the reference potential (V_{ref}) based on the electrode voltage and the first control signal; and
generating the second control signal based on the determined duration.

9. The method according to claim 1, wherein the first threshold voltage comprises a first value (th_cb_p) associated with residual positive charges and a second value (th_cb_n) associated with residual negative charges.

10. The method according to claim 2 or 3, wherein the second threshold voltage comprises a third value (th_sc_p) associated with a short circuit protection positive voltage and a fourth value (th_sc_n) associated with a short circuit protection negative voltage.

11. The method according to claim 5, wherein the third threshold voltage comprises a fifth value (th_ec_p) associated with an electrochemical-safe positive voltage and a sixth value (th_ec_n) associated with an electrochemical-safe negative voltage.

12. A method for controlling a stimulator (210), comprising:
obtaining a voltage of an electrode (110) coupled to the stimulator (210);
generating a first stimulus signal to control a drive circuit (211) of the stimulator (210) to apply a first electrical pulse to the electrode (110), wherein the first electrical pulse has a first charge polarity;
comparing the obtained electrode voltage with a second threshold voltage during the application of the first electrical pulse to the electrode (110); and
if magnitude of the electrode voltage is not less than magnitude of the second threshold voltage, disconnecting an electrical connection between a power supply (400) of the drive circuit (211) and the electrode (110) and generating a third control signal to connect the electrode (110) to a reference potential (V_{ref}).

13. The method according to claim 12, further comprising:
generating a second stimulus signal to control the drive circuit (211) to apply a second electrical pulse to the electrode (110), wherein the second electrical pulse has a second charge polarity that is opposite to the first charge polarity;
comparing the obtained electrode voltage with the second threshold voltage during the application of the second electrical pulse to the electrode (110); and
if the magnitude of the electrode voltage is not less than the magnitude of the second threshold voltage, disconnecting the electrical connection between the power supply (400) of the drive circuit (211) and the electrode (110) and generating the third control signal to connect the electrode (110) to the reference potential (V_{ref}).

14. The method according to claim 12 or 13, wherein the second threshold voltage comprises a third value (th_sc_p) associated with a short circuit protection positive voltage and a fourth value (th_sc_n) associated with a short circuit protection negative voltage.

15. The method according to claim 12 or 13, wherein the third control signal is used to connect the electrode (110) to the reference potential (V_{ref}) via a current limiting element (2113C).

16. A method for controlling a stimulator (210), comprising:
obtaining a voltage of an electrode (110) coupled to the stimulator (210);
generating a first stimulus signal to control the drive circuit (211) to apply a first electrical pulse to the electrode (110), wherein the first electrical pulse has a first charge polarity;
generating a second stimulus signal to control the drive circuit (211) to apply a second electrical pulse to the electrode (110), wherein the second electrical pulse has a second charge polarity that is opposite to the first charge polarity;
comparing the obtained electrode voltage with a third threshold voltage during a period between the application of the first electrical pulse and the application of the second electrical pulse; and
if magnitude of the electrode voltage is not less than magnitude of the third threshold voltage, generating an alert signal.

17. The method according to claim 16, wherein the third threshold voltage comprises a fifth value (th_ec_p) associated with an electrochemical-safe positive voltage and a sixth value (th_ec_n) associated with an electrochemical-safe negative voltage.

18. A stimulator (210), comprising:
a drive circuit (211), adapted to be coupled to an electrode (110);
a detection circuit (212), adapted to be coupled to the electrode (110) and configured to detect an electrode voltage; and
a controller (213), coupled to the drive circuit (211) and the detection circuit (212) and configured to:
obtain a voltage of the electrode (110);
compare the obtained electrode voltage with a first threshold voltage during a period when the stimulator (210) does not apply an electrical pulse to the electrode (110);
if magnitude of the electrode voltage is not less than magnitude of the first threshold voltage, generate a first control signal to control the drive circuit (211) to apply at least one electrical pulse to the electrode (110), wherein a charge polarity of the at least one electrical pulse is opposite to a charge polarity indicated by the electrode voltage; and
after applying the at least one electrical pulse, generate a second control signal to connect the electrode (110) to a reference potential (V_{ref}).

19. The stimulator (210) according to claim 18, wherein the controller (213) is further configured to:
generate a first stimulus signal to control the drive circuit (211) to apply a first electrical pulse to the electrode (110), wherein the first electrical pulse has a first charge polarity;
comparing the obtained electrode voltage with a second threshold voltage during the application of the first electrical pulse to the electrode (110); and
if the magnitude of the electrode voltage is not less than magnitude of the second threshold voltage, disconnect an electrical connection between a power supply (400) of the drive circuit (211) and the electrode (110) and generate a third control signal to connect the electrode (110) to the reference potential (V_{ref}).

20. The stimulator (210) according to claim 19, wherein the controller (213) is further configured to:
generate a second stimulus signal to control the drive circuit (211) to apply a second electrical pulse to the electrode (110), wherein the second electrical pulse has a second charge polarity that is opposite to the first charge polarity;
compare the obtained electrode voltage with the second threshold voltage during the application of the second electrical pulse to the electrode (110); and
if the magnitude of the electrode voltage is not less than the magnitude of the second threshold voltage, disconnect the electrical connection between the power supply (400) of the drive circuit (211) and the electrode (110) and generate the third control signal to connect the electrode (110) to the reference potential (V_{ref}).

21. The stimulator (210) according to claim 20, wherein the controller (213) is further configured to:
compare the obtained electrode voltage with a third threshold voltage during a period between the application of the first electrical pulse and the application of the second electrical pulse; and
if the magnitude of the electrode voltage is not less than magnitude of the third threshold voltage, generate an alert signal.

22. The stimulator (210) according to claim 21, wherein the controller (213) comprises a processing device (2131) and a comparator circuit (2132), wherein the comparator circuit (2132) comprises:
a first multiplexer (2132A), configured to output one of a first value (th_cb_p) associated with residual positive charges, a third value (th_sc_p) associated with a short circuit protection positive voltage, and a fifth value (th_ec_p) associated with an electrochemical-safe positive voltage;
a first comparator (2132B), configured to: compare the electrode voltage detected by the detection circuit (212) with a value (th_p) output by the first multiplexer (2132A), and provide a comparison result for the processing device (2131);
a second multiplexer (2132C), configured to output one of a second value (th_cb_n) associated with residual negative charges, a fourth value (th_sc_n) associated with a short circuit protection negative voltage, and a sixth value (th_ec_n) associated with an electrochemical-safe negative voltage; and
a second comparator (2132D), configured to: compare the electrode voltage detected by the detection circuit (212) with a value (th_n) output by the second multiplexer (2132C) and provide a comparison result for the processing device (2131).

23. A stimulator (210), comprising:
a drive circuit (211), adapted to be coupled to an electrode (110);
a detection circuit (212), adapted to be coupled to the electrode (110) and configured to detect an electrode voltage; and
a controller (213), coupled to the drive circuit (211) and the detection circuit (212) and configured to:
obtain a voltage of the electrode (110);
generate a first stimulus signal to control the drive circuit (211) to apply a first electrical pulse to the electrode (110), wherein the first electrical pulse has a first charge polarity;
compare the obtained electrode voltage with a second threshold voltage during the application of the first electrical pulse to the electrode (110); and
if magnitude of the electrode voltage is not less than magnitude of the second threshold voltage, disconnect an electrical connection between a power supply (400) of the drive circuit (211) and the electrode (110) and generate a third control signal to connect the electrode (110) to a reference potential (V_{ref}).

24. The stimulator (210) according to claim 23, wherein the controller (213) is further configured to:
generate a second stimulus signal to control the drive circuit (211) to apply a second electrical pulse to the electrode (110), wherein the second electrical pulse has a second charge polarity that is opposite to the first charge polarity;
compare the obtained electrode voltage with the second threshold voltage during the application of the second electrical pulse to the electrode (110); and
if the magnitude of the electrode voltage is not less than the magnitude of the second threshold voltage, disconnect the electrical connection between the power supply (400) of the drive circuit (211) and the electrode (110) and generate the third control signal to connect the electrode (110) to the reference potential (V_{ref}).

25. The stimulator (210) according to claim 23 or 24, wherein the third control signal is used to connect the electrode (110) to the reference potential (V_{ref}) via a current limiting element (2113C).

26. A stimulator (210), comprising:
a drive circuit (211), adapted to be coupled to an electrode (110);
a detection circuit (212), adapted to be coupled to the electrode (110) and configured to detect an electrode voltage; and
a controller (213), coupled to the drive circuit (211) and the detection circuit (212) and configured to:
obtain a voltage of the electrode (110);
generate a first stimulus signal to control the drive circuit (211) to apply a first electrical pulse to the electrode (110), wherein the first electrical pulse has a first charge polarity;
generate a second stimulus signal to control the drive circuit (211) to apply a second electrical pulse to the electrode (110), wherein the second electrical pulse has a second charge polarity that is opposite to the first charge polarity;
compare the obtained electrode voltage with a third threshold voltage during a period between the application of the first electrical pulse and the application of the second electrical pulse; and
if magnitude of the electrode voltage is not less than magnitude of the third threshold voltage, generate an alert signal.

27. The stimulator (210) according to any one of claims 18, 23 and 26, further comprising:
a passive protection component (214), coupled between the drive circuit (211) and the electrode (110), and resistance of the passive protection component (214) increases as a current flowing through the passive protection component (214) increases or a voltage crossing the passive protection component (214) increases.

28. The stimulator (210) according to claim 27, wherein the drive circuit (211) comprises a first current source (2111) coupled between a power supply potential and the electrode (110) and a second current source (2112) coupled between the ground potential and the electrode (110), and the passive protection component (214) is coupled between the first current source (2111) and the electrode (110).

29. The stimulator (210) according to claim 27, wherein the passive protection component (214) comprises a self-fuse component, and the self-fuse component is configured to fuse when the current flowing through the self-fuse component exceeds a fuse threshold.

30. The stimulator (210) according to any one of claims 18, 23 and 26, wherein the drive circuit (211) comprises a passive balancing branch (2113), the passive balancing branch (2113) comprises a first switch (2113A), a second switch (2113B), and a current limiting element (2113C), the first switch (2113A) and a parallel circuit formed by the second switch (2113B) and the current limiting element (2113C) are connected in series between the electrode (110) and the reference potential (V_{ref}), resistance of the current limiting element (2113C) is adjustable, and the controller (213) is configured to control the first switch (2113A) and the second switch (2113B) to turn on and off.

31. The stimulator (210) according to any one of claims 18, 23 and 26, wherein the detection circuit (212) comprises a plurality of voltage divider resistors or a plurality of voltage divider capacitors (Z₀ and Z₁) connected in series.

32. A brain-computer interface system (1000A and 1000B), comprising:
an electrode (110); and
the stimulator (210) according to any one of claims 18 to 31.

33. A chip (200), comprising:
a processor (220); and
the stimulator (210) according to any one of claims 18 to 31.
